# EUROPEAN PATENT SPECIFICATION

(11) **EP 2 416 818 B1**
(45) Date of publication and mention of the grant of the patent: **13.11.2019**
(21) Application number: 10711162.7
(22) Date of filing: 27.03.2010
(51) Int. Cl.: A61M 1/16, A61M 1/32, A61M 1/34

(54) **SYSTEM FOR THE TREATMENT OF BLOOD**
SYSTEM ZUR BEHANDLUNG VON BLUT
SYSTÈME POUR LE TRAITEMENT SANGUIN

(30) Priority: 09.04.2009 EP 09005223
(43) Date of publication of application: 15.02.2012
(73) Proprietor: Extra Corporeal Solutions S.r.l., 84132 Salerno (IT)
(72) Inventor: FARNIKOVA, Helena, 03049 Regensburg (DE)
(74) Representative: Mincone, Antimo
(86) International application number: PCT/EP2010/001959
(87) International publication number: WO 2010/115533

(56) References cited:
- EP-A1- 1 415 673
- US-A- 3 953 329
- US-A- 5 194 157
- US-A1- 2008 243 045

## Description

The present invention relates to a system for the treatment of blood. In particular, the invention concerns a highly efficient system for the safe removal of CO2 and bicarbonates from the blood.

During these last years the medical community recognized that Mechanical Ventilation in Intensive Care Units is a necessary but dangerous practice; in fact, even if often represents the only possibility to maintain the patient alive, it brings high risks and, if no properly settled, it is often the cause of Ventilatory Induced Lung Injury (VILI), Barotraumas, Pulmonary Fibrosis, etc.

For this reason some tentative solutions for patients with damaged lungs arose within the extracorporeal approach: the gas exchange, that usually occurs in the Alveoli, is obtained by means of a circuit in which the patient's blood meets a supplied oxygen flow in a component usually called oxygenator; in said component, for the differences in relative partial pressures, oxygen reaches the red cells in the blood stream and the CO2, that comes from the metabolic production, goes out. This approach would greatly help the correct setting of the ventilator machine within very protective parameters and, in some cases, it avoids the use of the Mechanical Invasive Ventilation allowing the patient to be treated with Non Invasive Ventilation or even with only spontaneous breathing. Several different approaches have been experimented.

A first attempt employs the difference in pressure between the arteries and veins to get the blood pass thru the oxygenator; but, since the arterial blood is relatively poorer in CO2 content and richer in O2, it is necessary an high flow to get the complete removal of CO2. To get this flow, a relatively large catheter must be inserted in the patient' vessels, both in an artery and in a vein (two accesses). The arterial blood that goes thru the oxygenator do not pass the natural regions of the body that therefore lack a proper blood supply. This solution generates a risk of ischemia of lower limbs. Furthermore, it naturally requires an arterial access (with risk of hemorrhage and contamination), needs a stable (or pharmacologically stabilized) arterial pressure and cardiac output.

Other systems use a venous-venous approach, thus requiring a pump driven system. This approach is easier and safer because there is no need for inserting a catheter in an artery. The treatment can be even less invasive reducing the blood flow below 500 ml/min, this often requires only one venous access point (with a double lumen catheter), with the only disadvantage represented by a reduced efficiency but without any dangerous side effects. At the moment, these mini-invasive systems do not exceed the removal of 50% of the metabolic production in CO2 and an improvement of this removal capability would be very advantageous in a extended area of application therapies.

US 2008/243045 A1 discloses a system for the treatment of blood, in particular for the removal of CO2 from the blood, comprising a hemofilter, provided with means for separating the plasmatic water from the blood, and a device for removing CO2.

It is an aim of the present invention to provide a system for a greater removal of CO2 and bicarbonates from the blood, in a safe way, acting on (but not limited to) the plasmatic water obtained from the blood. In other words, the CO2 and the bicarbonates are not removed directly from the whole blood, but from the plasmatic water which has been previously separated from the other more fragile components of the blood thanks to a Hemofilter (or similar device). This solution gives the possibility to treat only the fraction of blood that contains high percentage of bicarbonates with procedures that would damage the other components (red cells, white cells, platelets, etc.)

For a better comprehension of the new technique here described, it will be explained how gases are carried in the blood circulation.

Oxygen (02) is linked to hemoglobin, a protein inside the red cells. The quantity of oxygen that a certain amount of blood can carry is fixed: four molecules of oxygen per each molecule of hemoglobin. Under normal condition 100 ml of blood can carry 20.1 ml of oxygen (O2).

A different system is used for the transport of CO2: a percentage of about 70% (different studies give different values, these values must be considered as averages for explanation purposes) is converted in bicarbonate ions (HCO₃⁻) the bicarbonates are a sort of "concentrated" form of CO2 , about the 25% is carried as carbamine compounds, where CO2 is linked with proteins inside the red cells. Approx. the 5% is free, in solution.

The bicarbonate ions are in solution in the plasmatic water, outside the solid components of blood. The plasmatic water can be easily separated from blood with a standard hemofilter (for Dialysis or for C.R.R.T. Continuous Renal Replacement Therapy).

According to the present invention, the plasmatic water (also called ultrafiltrate) can be submitted to treatment that would damage the other blood components in whole blood; these treatments, which can be different and in combination of two or more, can reduce dramatically the content of bicarbonate ions thus reducing the total content of CO2 in the blood without any side effect on white and red cells.

After the treatment, the plasmatic water with reduced bicarbonate content is brought back and mixed with the blood flow before giving it back to the patient.

In this way, even with an extracorporeal circuit with a blood flow of less than 500 ml/min, it is possible to remove a bicarbonates content that corresponds to the total production of metabolic CO2, relieving the damaged lungs from the task of exchanging waste products.

According to the present invention, there is a system as claimed in independent claims 1 and 9.

Other features of the invention are described in the dependant claims.

The advantages of the invention will be more evident by the following description.

A non-limiting embodiment of the present invention will be described by way of example with reference to the accompanying drawings, in which:
- Figure 1 shows, schematically, a circuit relevant to a system for the treatment of the blood in accordance with the teachings of the present invention;
- Figure 2 shows another possible embodiment of the invention;
- Figure 3 shows a further embodiment of the invention;
- Figure 4 shows a possible embodiment of a component of the circuits shown in the Figures 1-3;
- Figures 5, 6, 7 show, schematically, three further embodiments of the invention.

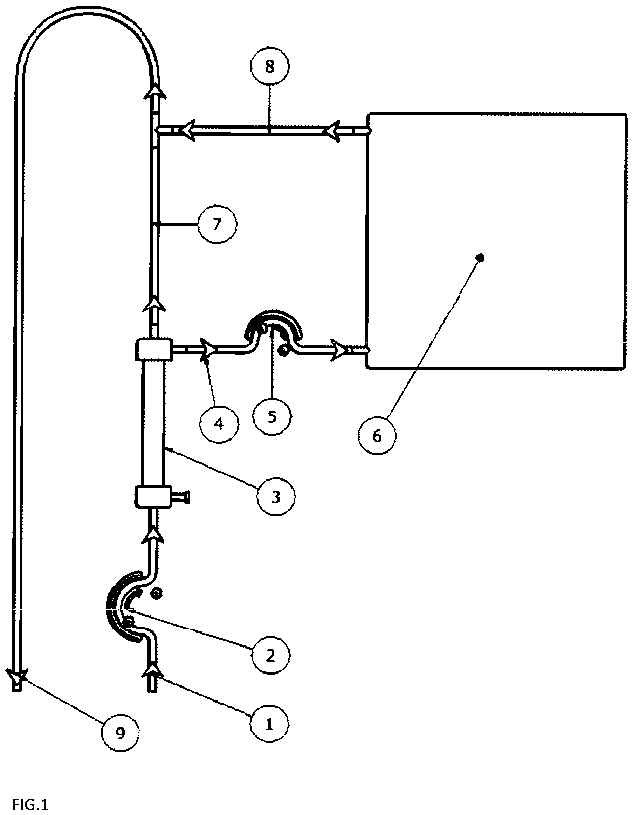

With reference to the enclosed drawings, the connection to the patient is of venous-venous type. In practice, the blood comes from and returns to a patient vein. A possible embodiment of a circuit in accordance with the invention has been explained referring to Figure1.

Using the reference numerals of the drawings, the blood comes from the patient's vein, usually the Jugular or the Femoral, by a tube line (1) drained by means of a first pump (2).

Downstream of the pump (2) is disposed a Hemofilter or a Dialyzer (3), in which is pushed the blood for separating the Plasmatic Water (which, downstream of the Hemofilter 3, passes in the tube line 4) and the solid components of blood (which, downstream of the Hemofilter 3, pass in the tube line 7).

The Plasmatic Water, which passes in the tube line (4), is drained by the pump (5), generally at a flow that is not greater than the 25% of the blood flow to avoid excess of fluidity loss in the remaining blood.

Downstream of the tube line (4) there is a device for removing bicarbonates and/or CO2, schematically represented by the block (6). In particular, the block (6) represents one (or a combination of more) of the possible methods for removing bicarbonates and/or CO2 which will be described below,. Downstream of the block (6), the treated Plasmatic Water is brought back to the main fraction of blood by a tube line (8), thus coming back to the original formula except for a great reduction in CO2/bicarbonates content. This blood is given back to the patient with the tube line (9),

According to the invention, it is very important the advantage of the venous-venous approach in terms of safety for the patient, easiness of treatment management and great reductions in side effects. The aim of the invention is to remove with a single catheter (double lumen) and with a low flow, the amount of the CO2 metabolic production close to the 100%.

This simple circuit has the added advantage of a low cost components set.

Advantageously, in order to achieve better results it will be possible to add, before or after the hemofilter of the circuit of Figure 1, an oxygenator which may help in removing the fraction of CO2 that is linked to proteins (25 - 30%) inside the red cells and the small percentage that is in solution (5-10%).

The presence of the Oxygenator (10) will also give a contribution to the patient's damaged lungs with the complete oxygenation of the portion of blood treated. The effect of the Oxygenator will also greatly compensates the pH modification produced by the bicarbonate removal bringing it back closer to the standard values.

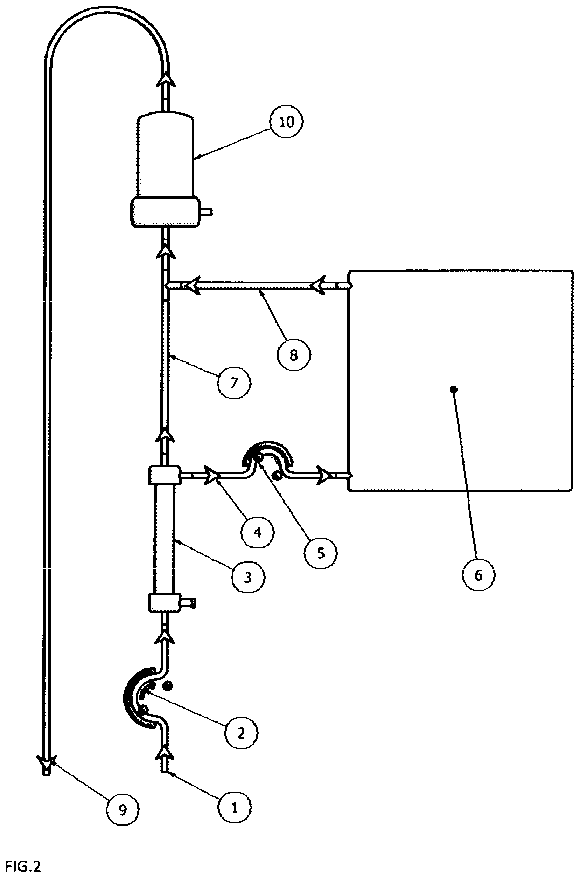

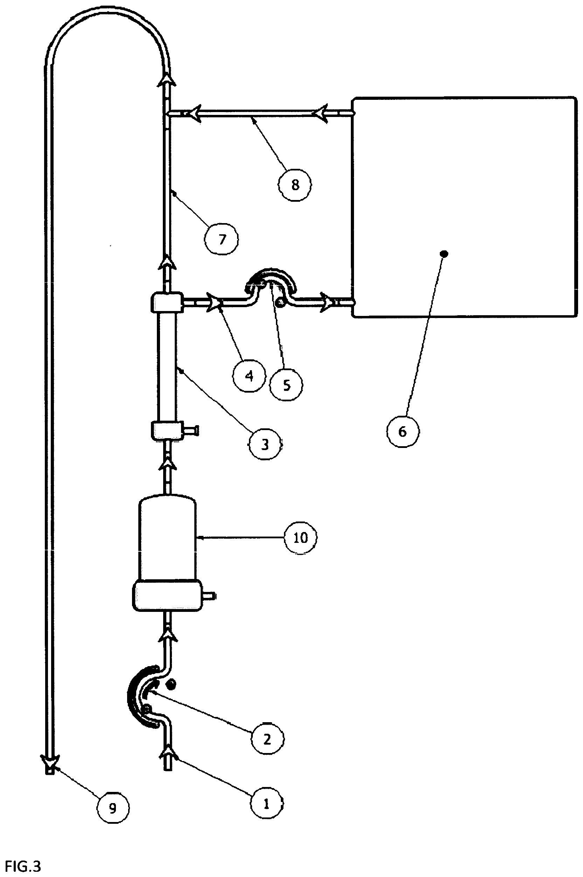

Two possible embodiments of a circuit including the oxygenator are shown in Figures 2 and 3.

In the example of Figure 2 the oxygenator (10) is disposed downstream of the Hemofilter (3) and downstream of the tube line (8), i.e. in a zone in which the purified plasmatic water is brought back to the untouched fraction of the blood. In this way, it is possible to increase the quantity of bicarbonate and CO2 removed.

In the example of Figure 3 the oxygenator (10) is disposed upstream of the Hemofilter (3). In this way, it is possible to obtain downstream of the same oxygenator, a blood (and a plasmatic water) having a fully percentage of oxygen.

The presence of oxygen may vary the equilibrium in the reaction that transforms CO2 in bicarbonates, in carbonic acid (that is a middle product) and in solution; thus, the choice of the best circuit will be influenced by this factor. In this position, furthermore, the blood pressure inside the Oxygenator will be increased by the resistance of the hemofilter to the blood flow, thus reducing the risk of direct passage of O2 in bubbles in the blood stream, that could bring to embolisms danger.

The block (6) represents one or more of the following sub-systems:

### A) Diffusion / convection

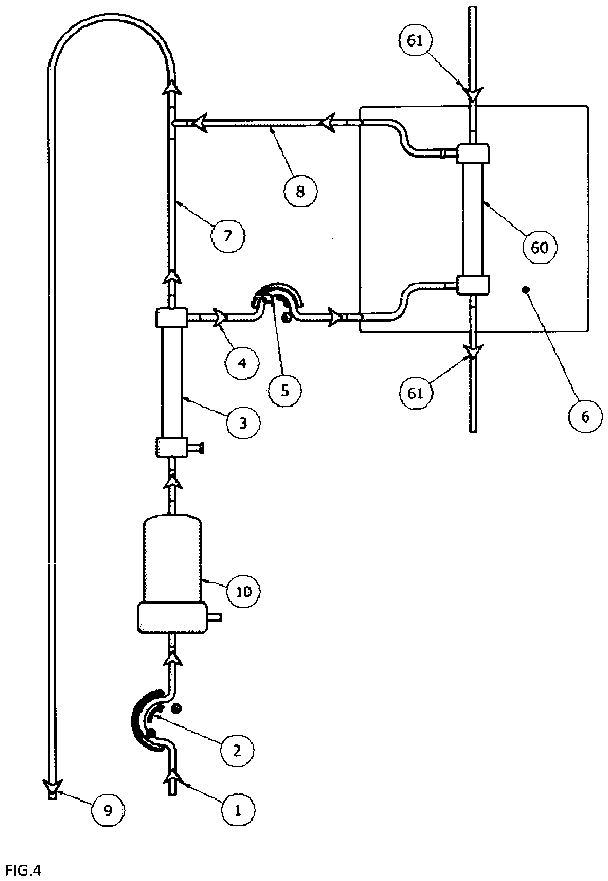

The plasmatic water is pushed in a haemofilter / haemoconcentrator where a semi-permeable membrane separates it from a similar solution (like dialysis solutions) with less or no bicarbonates. For the effect of the diffusion/convection, bicarbonates migrates thru the membrane and goes in the other solution, leaving the Plasmatic Water. This result can be easily obtained making the Plasmatic Water to pass thru a dialyzer filter (61) where the other side is washed with the proper solution. In Figure 4 is shown a possible example in which said similar solution passes thru the tube line (61), which, in turn, passes thru the dialyzer filter (60). In other words, the membrane is permeable by the plasmatic water; the membrane is not permeable by the protein and corpuscular parts of the blood. In practice, is the solute which passes thru the membrane.

### B) Heat

The Plasmatic Water is heated to a convenient temperature (about 90 °C). At this temperature the bicarbonates precipitate; the Plasmatic Water passes a standard convenient filter that holds the precipitate and after being cooled at 40°, it is mixed back with blood (this will also reduce the loss of temperature in the extracorporeal circuit as an added advantage). According to this example, the system comprises an heating device acting on the plasmatic water; in practice, the block (6) comprises a duct and/or a filter for the passage of the plasmatic water and one or more heating means apt to heat the same plasmatic water.

### C) Electrolysis

1) The plasmatic water goes under an electrolytic cell where positive H+ ions are delivered by an electrode. The bicarbonates in the plasmatic water will transform to CO2 increasing the CO2 partial pressure that can be eliminated in a standard bubble-trap chamber or in an oxygenator.
2) The proper current and electrodes will catalyze bicarbonate into the electrolytic cell, removing it from the water circulation and blood.

In this case, the system includes: an electrolytic cell (or similar device), means for eliminating the reaction products (filter).

### D) Precipitation

A container, filled with the proper material, (i.e. Calcium Gluconate) will make the bicarbonate in the Plasmatic Water to precipitate. A convenient filter will hold it inside the container. In this case, the system includes a container filled with a precipitating substance which determine the precipitation of the bicarbonate, filtering means for collecting the residual products.

### F) Chemical transformation

The plasmatic water will pass thru a container filled with Barium Hydroxide pellets that would react with bicarbonate forming Barium Carbonate, water and hydroxyl ions. Other chemical products could be used with the same objective, i.e. for forming a Carbonate reacting with the bicarbonate contained in the plasmatic water. In this case, the system includes a container filled with Barium Hydroxide pellets or other similar chemical products.

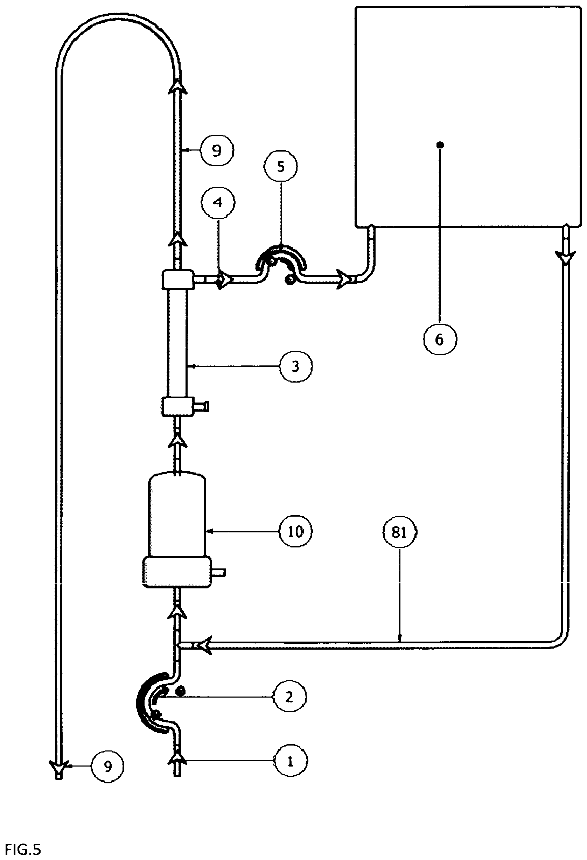

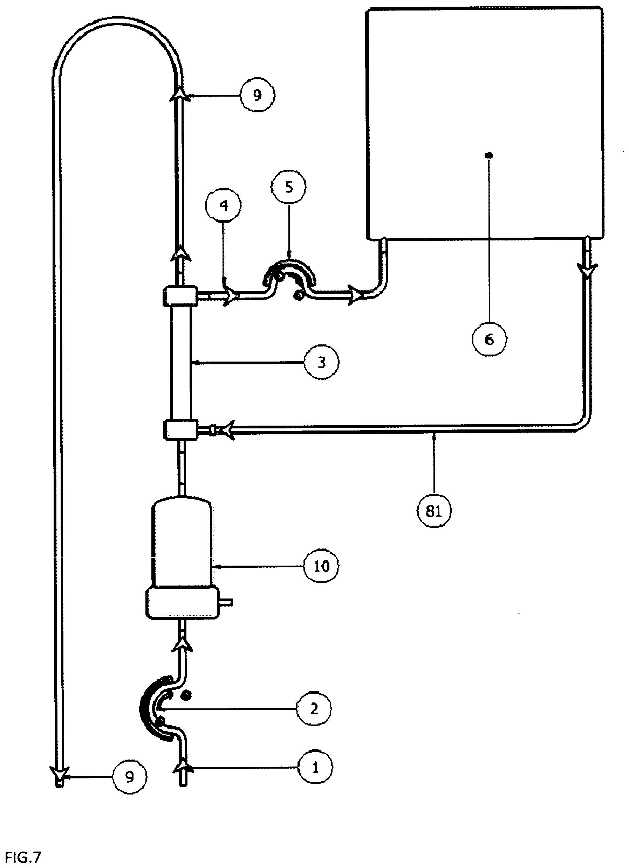

In the embodiments of the invention shown in the drawings of Figures 5-7, the output of the device (6) is connected to the blood circuit upstream of the hemofilter (3) or in correspondence of the same hemofilter (Figure 7).

In these cases the system comprises an oxygenator.

According to the example of Fig.5, a third line tube (81) connects the device (6) to the blood circuit upstream of the oxygenator (10), which, in turn, is disposed upstream of the hemofilter (3) and downstream of the pump (2). In practice, the third line tube (8) is connected to the blood circuit in a point disposed between the pump (2) and the oxygenator (10).

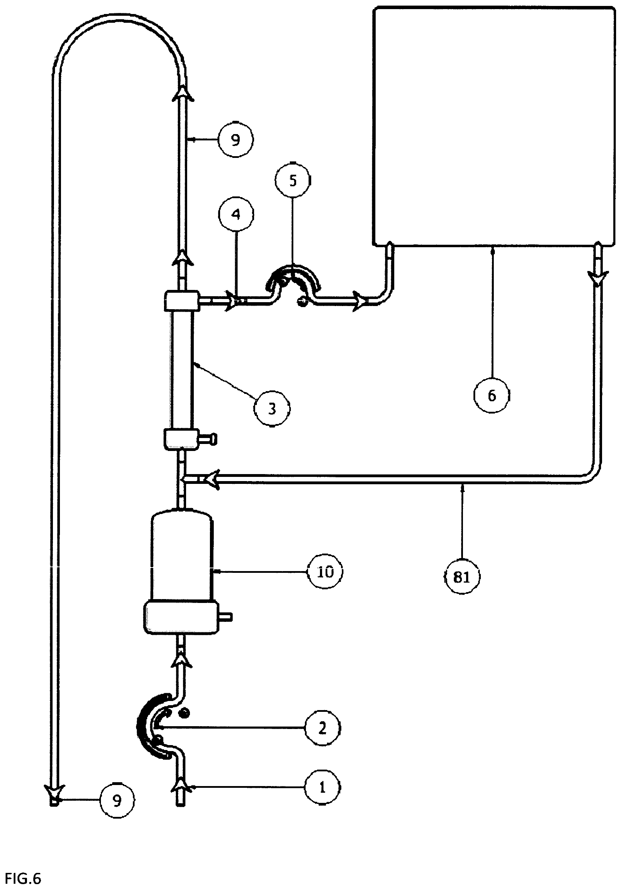

In the example of Fig.6, the third line tube (81) connects the device (6) to the blood circuit upstream of the hemofilter (3) and downstream of the oxygenator (10).

In the example of Fig.7, the third line tube (81) connects the device (6) to the blood circuit directly to one of the inlets of the hemofilter (3); in practice, the tube line (8) is connected to the upstream inlet of the hemofilter (3) which in the other examples is free.

The system will also include the possibility for injection of anticoagulants (like Heparine or Calcium-Cytrate solutions as commonly used during standard extracorporeal treatments.

Clearly, changes may be made to the blood treatment machine and unit as described and illustrated herein without, however, departing from the scope of the present invention.

## Claims

1. System for the treatment of blood, in particular for the safe removal of CO2 and Bicarbonates from the blood, in a blood circuit connectable to a patient by means of a venous-venous connection having at least an inlet or tube line (1) for receiving a flow of blood for CO2 or bicarbonate removal and an outlet or tube line (9) for the blood deprived of CO2 or bicarbonate, and a hemofilter or dialyzer (3), disposed between said inlet (1) and said outlet (9), and provided with means for separating the plasmatic water from the blood and for conveying the same plasmatic water through a second tube line (4); **characterized in that** it comprises a device (6) for removing bicarbonates and/or CO2, disposed and acting on said second tube line (4), in a position which is downstream of said hemofilter (3) and upstream of a third tube line (8) which brings back the treated plasmatic water in the blood circuit downstream of said hemofilter (3).

2. System according to claim 1, **characterized in that** said device (6) comprises an osmotic membrane which, for the osmotic effect, determinates the migration of the bicarbonates through the membrane, leaving the plasmatic water.

3. System according to claim 1, **characterized in that** said device (6) comprises a heating device acting on the plasmatic water.

4. System according to claim 1, **characterized in that** said device (6) comprises: an electrolytic cell and means for eliminating the relevant gas or precipitates formed by the reaction with the plasmatic water.

5. System according to claim 1, **characterized in that** said device (6) comprises a container filled with a precipitating substance which determine the precipitation of the bicarbonate, filtering means for collecting the residual products.

6. System according to claim 1, **characterized in that** said device (6) comprises a container filled with a Barium Hydroxide pellets or with another similar substance suitable for reacting with bicarbonate contained in plasmatic water.

7. System according to one of the preceding claims, **characterized in that** said device (6) comprises an oxygenator (10) disposed upstream of the hemofilter (3).

8. System according to one of the preceding claims from 1 to 6, **characterized in that** said device (6) comprises an oxygenator (10) disposed downstream of the hemofilter (3).

9. System for the treatment of blood, in particular for the safe removal of CO2 and Bicarbonates from the blood, in a blood circuit connectable to a patient by means of a venous-venous connection having at least an inlet or tube line (1) for receiving a flow of blood for CO2 or bicarbonate removal and an outlet or tube line (9) for the blood deprived of CO2 or bicarbonate, comprising:
- a hemofilter or dialyzer (3), disposed between said inlet (1) and said outlet (9), and provided with means for separating the plasmatic water from the blood and for conveying the same plasmatic water through a second tube line (4);
- an oxygenator (10) disposed upstream of the hemofilter (3);
**characterized in that** it comprises:
- a device (6) for removing bicarbonates and/or CO2, disposed and acting on said second tube line (4), in a position which is downstream of said hemofilter (3);
- a third tube line (81) which brings back the treated plasmatic water in the blood circuit upstream of said hemofilter or in an inlet of the same hemofilter (3) disposed upstream.

10. System according to claim 9, **characterized in that** third tube line (8) is connected to the blood circuit upstream of the hemofilter (3) and downstream of the oxygenator (10).

11. System according to claim 9, **characterized in that** third tube line (8) is connected to the blood circuit upstream of the hemofilter (3) and of the oxygenator(10).

12. System according to one of the preceding claims, **characterized in that** said device (6) comprises a first pump (2) disposed upstream of the hemofilter (3).

13. System according to one of the preceding claims, **characterized in that** said device (6) comprises a second pump (5) disposed on said second tube line (4).

## Patentansprüche

1. System zur Behandlung von Blut, insbesondere zur sicheren Entfernung von CO₂ und Bicarbonaten aus dem Blut, in einem Blutkreislauf, der mit einem Patienten über eine venös-venöse Verbindung verbindbar ist, mit mindestens einer Einlass- oder Schlauchleitung (1) zur Aufnahme eines Blutstroms zur CO₂- oder Bicarbonatentfernung und einer Auslass- oder Schlauchleitung (9) für das von CO₂ oder Bicarbonat befreite Blut, und einem Hämofilter oder Dialysator (3), der zwischen dem Einlass (1) und dem Auslass (9) angeordnet ist und mit Mitteln zum Trennen des plasmatischen Wassers vom Blut und zum Fördern des gleichen plasmatischen Wassers durch eine zweite Schlauchleitung (4) versehen ist;
**dadurch gekennzeichnet, dass** es
eine Vorrichtung (6) zum Entfernen von Bicarbonaten und/oder CO₂ umfasst, die auf der zweiten Schlauchleitung (4) in einer Position angeordnet ist und darauf wirkt, die stromabwärts des Hämofilters (3) und stromaufwärts einer dritten Schlauchleitung (8) liegt, die das behandelte plasmatische Wasser in dem Blutkreislauf stromabwärts des Hämofilters (3) zurückführt.

2. System nach Anspruch 1, **dadurch gekennzeichnet, dass** die Vorrichtung (6) eine osmotische Membran umfasst, die für den osmotischen Effekt die Migration der Bicarbonate durch die Membran bestimmt, die das plasmatische Wasser verlassen.

3. System nach Anspruch 1, **dadurch gekennzeichnet, dass** die Vorrichtung (6) eine Heizvorrichtung umfasst, die auf das plasmatische Wasser wirkt.

4. System nach Anspruch 1, **dadurch gekennzeichnet, dass** die Vorrichtung (6) umfasst: eine Elektrolysezelle und Mittel zum Eliminieren des relevanten Gases oder der Niederschläge, die durch die Reaktion mit dem plasmatischen Wasser gebildet werden.

5. System nach Anspruch 1, **dadurch gekennzeichnet, dass** die Vorrichtung (6) einen Behälter, der mit einer Fällungssubstanz gefüllt ist, die die Fällung des Bicarbonats bestimmt, Filtermittel zum Sammeln der Restprodukte umfasst.

6. System nach Anspruch 1, **dadurch gekennzeichnet, dass** die Vorrichtung (6) einen Behälter umfasst, der mit Bariumhydroxid-Pellets oder mit einer anderen ähnlichen Substanz gefüllt ist, die zur Reaktion mit in plasmatischem Wasser enthaltenem Bicarbonat geeignet ist.

7. System nach einem der vorstehenden Ansprüche, **dadurch gekennzeichnet, dass** die Vorrichtung (6) einen Oxygenator (10) umfasst, der stromaufwärts des Hämofilters (3) angeordnet ist.

8. System nach einem der vorstehenden Ansprüche 1 bis 6, **dadurch gekennzeichnet, dass** die Vorrichtung (6) einen Oxygenator (10) umfasst, der stromabwärts des Hämofilters (3) angeordnet ist.

9. System zur Behandlung von Blut, insbesondere zur sicheren Entfernung von CO₂ und Bicarbonaten aus dem Blut, in einem Blutkreislauf, der mit einem Patienten über eine venös-venöse Verbindung verbindbar ist, mit mindestens einer Einlass- oder Schlauchleitung (1) zur Aufnahme eines Blutstroms zur CO₂- oder Bicarbonatentfernung und einer Auslass- oder Schlauchleitung (9) für das von CO₂ oder Bicarbonat befreite Blut, umfassend:
- einen Hämofilter oder Dialysator (3), der zwischen dem Einlass (1) und dem Auslass (9) angeordnet ist und mit Mitteln zum Trennen des plasmatischen Wassers vom Blut und zum Fördern des gleichen plasmatischen Wassers durch eine zweite Schlauchleitung (4) versehen ist;
- einen Oxygenator (10), der stromaufwärts des Hämofilters (3) angeordnet ist;
**dadurch gekennzeichnet, dass** es umfasst:
- eine Vorrichtung (6) zum Entfernen von Bicarbonaten und/oder CO₂, die auf der zweiten Schlauchleitung (4) in einer Position angeordnet ist und darauf wirkt, die stromabwärts des Hämofilters (3) liegt;
- eine dritte Schlauchleitung (81), die das behandelte plasmatische Wasser im Blutkreislauf stromaufwärts des Hämofilters oder in einem Einlass desselben stromaufwärts angeordneten Hämofilters (3) zurückführt.

10. System nach Anspruch 9, **dadurch gekennzeichnet, dass** die dritte Schlauchleitung (8) mit dem Blutkreislauf stromaufwärts des Hämofilters (3) und stromabwärts des Oxygenators (10) verbunden ist.

11. System nach Anspruch 9, **dadurch gekennzeichnet, dass** die dritte Schlauchleitung (8) mit dem Blutkreislauf stromaufwärts des Hämofilters (3) und des Oxygenators (10) verbunden ist.

12. System nach einem der vorstehenden Ansprüche, **dadurch gekennzeichnet, dass** die Vorrichtung (6) eine erste Pumpe (2) umfasst, die stromaufwärts des Hämofilters (3) angeordnet ist.

13. System nach einem der vorstehenden Ansprüche, **dadurch gekennzeichnet, dass** die Vorrichtung (6) eine zweite Pumpe (5) umfasst, die auf der zweiten Schlauchleitung (4) angeordnet ist.

## Revendications

1. Système de traitement du sang, en particulier pour une élimination en toute sécurité de CO₂ et de bicarbonates à partir du sang, dans un circuit sanguin pouvant être raccordé à un patient au moyen d'un raccord veineux-veineux ayant au moins une entrée ou conduite tubulaire (1) pour recevoir un flux sanguin pour une élimination de CO₂ ou de bicarbonate et une sortie ou une conduite tubulaire (9) pour le sang dépourvu de CO₂ ou de bicarbonate, et un hémofiltre ou dialyseur (3), disposé entre ladite entrée (1) et ladite sortie (9), et pourvu de moyens de séparation de l'eau plasmatique à partir du sang et de transport de cette eau plasmatique par l'intermédiaire d'une deuxième conduite tubulaire (4) ;
**caractérisé en ce qu'**il comprend
un dispositif (6) d'élimination de bicarbonates et/ou de CO₂, disposé et agissant sur ladite deuxième conduite tubulaire (4), en une position qui est en aval dudit hémofiltre (3) et en amont d'une troisième conduite tubulaire (8) qui fait revenir l'eau plasmatique traitée dans le circuit sanguin en aval dudit hémofiltre (3).

2. Système selon la revendication 1, **caractérisé en ce que** ledit dispositif (6) comprend une membrane osmotique qui, pour l'effet osmotique, détermine la migration des bicarbonates à travers la membrane, en laissant l'eau plasmatique.

3. Système selon la revendication 1, **caractérisé en ce que** ledit dispositif (6) comprend un dispositif chauffant agissant sur l'eau plasmatique.

4. Système selon la revendication 1, **caractérisé en ce que** ledit dispositif (6) comprend : une cellule électrolytique et des moyens d'élimination des gaz ou précipités importants formés par la réaction avec l'eau plasmatique.

5. Système selon la revendication 1, **caractérisé en ce que** ledit dispositif (6) comprend un récipient rempli d'une substance de précipitation qui détermine la précipitation du bicarbonate, et des moyens de filtration pour recueillir les produits résiduels.

6. Système selon la revendication 1, **caractérisé en ce que** ledit dispositif (6) comprend un récipient rempli de granulés d'hydroxyde barium ou d'une autre substance similaire appropriés pour réagir avec le bicarbonate contenu dans l'eau plasmatique.

7. Système selon l'une quelconque des revendications précédentes, **caractérisé en ce que** ledit dispositif (6) comprend un oxygénateur (10) disposé en amont de l'hémofiltre (3).

8. Système selon l'une quelconque des revendications 1 à 6, **caractérisé en ce que** ledit dispositif (6) comprend un oxygénateur (10) disposé en aval de l'hémofiltre (3).

9. Système de traitement du sang, en particulier pour une élimination en toute sécurité de CO₂ et de bicarbonates à partir du sang, dans un circuit sanguin pouvant être raccordé à un patient au moyen d'un raccord veineux-veineux ayant au moins une entrée ou conduite tubulaire (1) pour recevoir un flux sanguin pour une élémination de CO₂ ou de bicarbonate et une sortie ou conduite tubulaire (9) pour le sang dépourvu de CO₂ ou de bicarbonate, comprenant :
- un hémofiltre ou dialyseur (3), disposé entre ladite entrée (1) et ladite sortie (3), et pourvu de moyens de séparation de l'eau plasmatique à partir du sang et de transport de cette eau plasmatique par l'intermédiaire d'une deuxième conduite tubulaire (4) ;
- un oxygénateur (10) disposé en amont de l'hémofiltre (3) ;
**caractérisé en ce qu'**il comprend :
- un dispositif (6) d'élimination de bicarbonates et/ou de CO2, disposé et agissant sur ladite deuxième conduite tubulaire (4), en une position qui est en aval dudit hémofiltre (3) ;
- une troisième conduite tubulaire (81) qui fait revenir l'eau plasmatique traitée dans le cicuit sanguin en aval dudit hémofiltre ou à une entrée de cet hémofiltre (3) disposé en amont.

10. Système selon la revendication 9, **caractérisé en ce qu'**une troisième conduite tubulaire (8) est raccordée au circuit sanguin en amont de l'hémofiltre (3) et en aval de l'oxygénateur (10).

11. Système selon la revendication 9, **caractérisé en ce qu'**une troisième conduite tubulaire (8) est raccordée au circuit sanguin en amont de l'hémofiltre (3) et de l'oxygénateur (10).

12. Système selon l'une quelconque des revendications précédentes, **caractérisé en ce que** ledit dispositif (6) comprend une première pompe (2) disposée en amont de l'hémofiltre (3).

13. Système selon l'une quelconque des revendications précédentes, **caractérisé en ce que** ledit dispositif (6) comprend une seconde pompe (5) disposée sur ladite deuxième conduite tubulaire (4).
